(19) Europäisches Patentamt

European Patent Office

Office européen des brevets

(11) Veröffentlichungsnummer: **0 290 986 B1**

(12) **EUROPÄISCHE PATENTSCHRIFT**

(45) Veröffentlichungstag der Patentschrift: **23.06.93**

(21) Anmeldenummer: **88107372.0**

(22) Anmeldetag: **07.05.88**

(51) Int. Cl.⁵: **C12N 15/31**, C12N 15/54, C12N 9/10

(54) **Resistenzgen gegen Phosphinothricin.**

(30) Priorität: **15.05.87 DE 3716309**

(43) Veröffentlichungstag der Anmeldung:
**17.11.88 Patentblatt 88/46**

(45) Bekanntmachung des Hinweises auf die
Patenterteilung:
**23.06.93 Patentblatt 93/25**

(84) Benannte Vertragsstaaten:
**CH DE ES FR GB IT LI NL**

(56) Entgegenhaltungen:
**EP-A- 0 173 327**
**EP-A- 0 239 801**
**EP-A- 0 257 542**

**Frankturter Allgemeine Zeitung, 4.Februar
1987, Beilage Natur und Wissenschaft, S. 29**

**Chemical Abstracts, Band 102, Nr. 17,
29.April 1985, Columbus, Ohio, USA. G. Donn
et al "Herbicide-resistant alfalfa cells : an
example of gene amplification in plants", S.
140, Zusammenfassung Nr. 144025**

**Helvetica Chimica Acta, Band 55, Fasc. 1,
1972 (Basel, Schweiz) E. Bayer et al**

**"Stoffwechselprodukte von Microorganismen ; Phosphinothricin und
Phosphinothricyl-Alanyl-Alanin", S. 224-239**

**"Plant gene systems and their biology", Vaeck et al., Proc. of a CIBA-Geigy-UCLA Symposium, Tammarron, Colorado, 2-8.02.1987,
p. 171-181, Alan R. Lin, Inc. US**

(73) Patentinhaber: **HOECHST AKTIENGESELL-
SCHAFT
Postfach 80 03 20
W-6230 Frankturt am Main 80(DE)**

(72) Erfinder: **Brauer, Dieter, Dr.
Berliner Strasse 56
W-6093 Flörsheim am Main(DE)**
Erfinder: **Bartsch, Klaus, Dr.
Memelstrasse 2
W-6233 Kelkheim (Taunus)(DE)**
Erfinder: **Donn, Günter, Dr.
Sachsenring 35
W-6238 Hofheim am Taunus(DE)**

EP 0 290 986 B1

## Beschreibung

Phosphinothricin (PTC, 2-Amino-4-methylphosphino-buttersäure) ist ein Glutaminsynthetase-Inhibitor. PTC ist ein "Baustein" des Antibiotikums Phosphinothricyl-alanyl-alanin. Dieses Tripeptid (PTT) ist aktiv gegen Gram-positive und Gram-negative Bakterien und auch gegen den Pilz Botrytis cinerea (Bayer et al., Helv. chim. Acta 55(1972)224). PTT wird von dem Stamm Streptomyces viridochromogenes Tü 494 (DMS 40736) produziert.

Die europäische Patentanmeldung mit der Veröffentlichungsnummer (EP-A) 0 173 327 bezieht sich auf die Biosynthese von PTT. In der Figur 7 ist ein nicht näher charakterisiertes Resistenzgen aus S. hygroscopicus FERM BP-130 (ATCC 21705) aufgeführt.

In der Frankfurter Allgemeinen Zeitung vom 4. Februar 1987, S. 29 (Beilage Natur und Wissenschaft, linke Spalte oben) wird berichtet, daß aus Bodenbakterien der Gattung Streptomyces ein Gen isoliert werden konnte, das für den Abbau von PTC verantwortlich ist.

In der nicht vorveröffentlichten europäischen Patentanmeldung (EP-A) 0 257 542 ist ein Resistenzgen gegen PTC vorgeschlagen, das aus dem obengenannten PTT-Produzenten S. viridochromogenes DSM 40736 stammt. Vaeck et al. (Plant Gene Systems and their Biology, Proceedings of a Ciba-Geigy-UCLA Symposium, Tamarron, Colorado, 2-8.2.1987, p. 171-181, Alan R. Liss, US) beschreiben die Klonierung eines Resistenzgens aus S. hygroscopicus.

Sowohl hinsichtlich der Produktion von PTC bzw. PTT als auch der Resistenz gegen diese Verbindung sind bisher nur Streptomyceten beschrieben bzw. vorgeschlagen worden.

Die große Gattung der pilzähnlichen Streptomyceten spielt unter den Bakterien in mehreren Beziehungen eine Sonderrolle: Streptomyceten stellen die bedeutendsten Produzenten von Antibiotika dar, sie bilden ein Mycel, das auch in der alternden Kolonie erhalten bleibt und häufig ein stark ausgebildetes Luftmycel entwickelt, und in gentechnischer Beziehung ist von Bedeutung, daß in ihrer DNA das Verhältnis A und T zu G und C etwa 30 zu 70 beträgt.

Überraschenderweise wurde nun gefunden, daß auch andere Bakterien PTC-Resistenz aufweisen können, insbesondere nicht-pilzähnliche, vorzugsweise Gram-negative Bakterien.

Aus der deutschen Patentschrift 2 717 440 ist es bekannt, daß PTC als nicht-selektives oder Total-Herbizid wirkt. Es zeichnet sich durch eine kurze biologische Halbwertszeit im Erdreich aus, d.h. dieses Herbizid wird im Boden durch Mikroorganismen offenbar sehr schnell modifiziert oder abgebaut, wobei die herbizide Wirkung verloren geht. Es gelang, aus Bodenproben, die aus PTC-behandelten Ackerböden stammten, PTC-modifizierende Mikroorganismen zu isolieren. Weiterhin konnten Bakterien aus Klärschlamm auf PTC-Resistenz selektiert werden, vorteilhaft nach der Behandlung mit Mutagenen.

Die Erfindung betrifft ein Resistenzgen gegen Phosphinothricin (PTC), erhältlich durch Selektion nicht-pilzähnlicher Bakterien auf PTC-Resistenz, Gewinnung der DNA, Anlegen einer Genbank, Isolierung PTC-resistenter Klone und Gewinnung des PTC-Resistenzgens aus diesen Klonen.

Die Erfindung betrifft vor allem ein neues Resistenzgen gegen PTC, welches aus nicht-pilzähnlichen Bakterien gewonnen wurde, dieses Gen enthaltende Zellen, insbesondere Pflanzenzellen, sowie dieses Gen enthaltende PTC-resistente Pflanzen. Ein weiterer Aspekt der Erfindung betrifft die Verwendung des Gens als Marker in Bakterien-und Pflanzenzellen. Weitere Aspekte der Erfindung sowie bevorzugte Ausgestaltungen werden im folgenden näher erläutert.

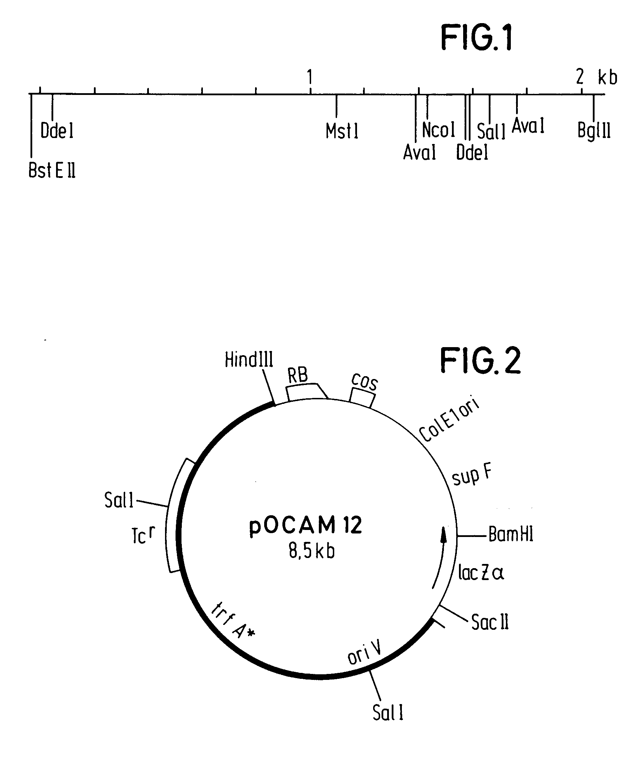

Das erfindungsgemäße Gen ist durch die Restriktionskarte gemäß Fig. 1 charakterisiert.

Die Fig. 2 zeigt den Vektor pOCAM 12, der bei der Erstellung einer Genbank zur Isolierung des Gens nach Fig. 1 dienen kann.

Die Selektion der PTC-resistenten nicht-pilzähnlichen Bakterien ergibt neben nicht-charakterisierten Mikroorganismen im wesentlichen nicht-sporenbildende gram-negative Stäbchen und zwar aerobe Organismen der Gattung Pseudomonas und Pseudomonaden-ähnliche Bakterien der Gattungen Alcaligenes und Agrobacterium sowie die fakultativ anaeroben Enterobakterien der Gattungen Enterobacter und Serratia sowie Bakterien der Gattung Cedecea.

Für die Zwecke der Erfindung ist eine genaue Charakterisierung des PTC-resistenten Bakteriums nicht erforderlich. Es genügt, wenn es in dem Selektionsmedium, das mit PTC angereichert ist, hinreichend wächst.

Nach der Charakterisierung geeigneter Gattungen und Arten kann man auch auf die in den Hinterlegungsstellen verfügbaren, wohlcharakterisierten Stämme der genannten Arten und Gattungen zurückgreifen und hieraus PTC-resistente Stämme selektieren.

Näher charakterisiert wurden Stämme der Arten Alcaligenes faecalis und eutrophus, Pseudomonas paucimobilis, Enterobacter agglomerans, Serratia plymuthica, Agrobacterium tumefaciens und Cedecea Gr. V. Ausgehend von dieser Erkenntnis kann man beispielsweise die bei der Deutschen Sammlung von Mikroorganismen unter den Nummern DSM 975 oder 30030 zugänglichen Alcaligenes-Stämme einsetzen.

Derartige PTC-resistente Bakterien bzw. durch Einführen des erfindungsgemäßen Gens PTC-resistent gemachte Mikroorganismen können auch in Kläranlagen verwendet werden, um PTC und dessen Derivate in Produktionsrückständen oder Abwässern abzubauen oder so zu modifizieren, daß ihr Abbau durch die üblichen Mikroorganismen-Populationen in Kläranlagen erfolgt.

Entsprechend dem Vorschlag in der EP-A 0 257 542 wurde das für die Inaktivierung von PTC verantwortliche Enzym als Glutaminsäure-N-acetyltransferase charakterisiert. Das für diese Aktivität verantwortliche Gen wurde isoliert und durch die Restriktionskarte gemäß Figur 1 und die DNA-Sequenz nach Tabelle 1 definiert. Diese DNA-Sequenz kann natürlich auch nach einem der bekannten Verfahren, beispielsweise nach der Phosphit-Methode, chemisch synthetisiert werden, wobei in an sich bekannter Weise auch modifizierte Gene hergestellt werden können.

Das Gen kann nach Fusion mit geeigneten, wirtsspezifischen Regulationselementen anderen, an sich PTC-sensitiven Organismen Resistenz gegen PTC vermitteln. Diese Resistenz kann als Selektionsmarker oder aber zur Gewinnung PTC-resistenter Nutzpflanzen dienen.

Bei der Behandlung dieser Nutzpflanzen mit PTC tritt dann nicht nur die Wirkung ein, daß unerwünschter Pflanzenwuchs gehemmt wird, sondern es werden auch unerwünschte Mikroorganismen auf den Nutzpflanzen reduziert.

In den folgenden Beispielen beziehen sich Prozentangaben auf das Gewicht, sofern keine anderen Angaben gemacht sind.

Beispiel 1:

"Screening" auf Minimalmedien und Modifikationstest

Die Bakterien werden mit LB-Medium (10 g Difco-Bacto-Trypton, 5 g Difco-Bacto-Hefeextrakt und 10 g Kochsalz/l) aus den Bodenproben extrahiert, über Nacht angezüchtet, mit Puffer (10 mM $Na_2HPO_4$/10 mM NaCl) gewaschen und auf Selektionsmedium der folgenden Zusammensetzung ausplattiert:
0,4 g NaCl, 0,8 g $KH_2PO_4$, 1,6 g $Na_2HPO_4$, 1,6 g D,L-PTC($NH_4$),
4 ml Glukose (10 %), 0,8 ml l M $MgSO_4$,
Wasser auf 800 ml;
bei Festmedien wird 1,4 % Agar zugegeben.

Zum Modifikationstest werden etwa 5 ml Bakteriensuspension auf ein Volumen von 200 $\mu$l reduziert und durch Zugabe von 5 $\mu$g Lysozym lysiert. Nach weiterer Zugabe von 1 $\mu$Ci 3,4-$^{14}$C-PTC wird ca. 4 h bei 28 °C inkubiert. Zur Analyse inkubiert man weitere 10 min bei 95 °C, kühlt in Eis ab und zentrifugiert 10 min in einer Tischzentrifuge. Vom klarem Überstand werden 10 $\mu$l-Volumina auf Cellulose-Dünnschichtplatten (Firma Merck, Darmstadt, Cellulose F, DC Alufolien) aufgetragen, aufsteigend chromatographiert (Laufmittel: Pyridin, n-Butanol, Essigsäure, Wasser im Volumenverhältnis 50 : 75 : 15 : 60), getrocknet und autoradiographiert. PTC hat unter diesen Bedingungen einen Rf-Wert von etwa 0,31 und N-Acetyl-PTC von etwa 0,33.

Zur Untersuchung einer Cosmid-Bank werden 5 Klone jeweils in 5 ml Medium angezogen, vereint, auf ein Volumen von etwa 300 $\mu$l reduziert und wie oben beschrieben aufgearbeitet.

Beispiel 2:

Herstellung des Cosmid-Vektors pOCAM 12

Der Cosmid-Vektor pOCAM 12 weist wegen seines ColE1-Replikationsursprungs in E. coli eine hohe Kopienzahl auf, hat den breiten Wirtsbereich des natürlich vorkommenden Plasmids RK2 sowie ein Resistenzgen gegen Tetracyclin. Der Vektor ist ein Abkömmling des mobilisierbaren Vektors pRK404 (G. Ditta et al., Plasmid 13 (1985) 149-153). Er kann nach Aufnahme von DNA-Fragmenten der Größe von 25 bis 40 kb in λ-Phagen verpackt werden. Dieser Vektor ist wie folgt erhältlich:

Der Vektor pTJS75 (T. S. Schmidhauser u. D. R. Helinski, J. Bacteriology 164 (1985) 446-455) wird mit HindIII geöffnet und die überhängenden Sequenzen durch Auffüllen mit DNA-Polymerase I (Klenow-Fragment) stumpfendig gemacht. Der Vektor pSDL 12 (A. Levinson et al., J. Molec. Appl. Genetics 2 (1984) 507-517) wird mit NaeI und SspI geöffnet und das große Fragment isoliert. Dieser und das stumpfendig gemachte linearisierte Plasmid pTJS75 werden nun mit einem stumpfendigen BglII-Segment aus pHC79 ligiert, welches die cos-Region enthält. Die 23 bp lange "Right Border" (RB) des Ti-Plasmids wurde synthetisiert und durch stumpfendige Ligation eingesetzt. Das gewünschte Plasmid pOCAM 12 von 8,5 kb Größe, das in der Fig. 2 dargestellt ist, wird durch Restriktionsanalyse charakterisiert.

Selbstverständlich kann für die Erstellung der Genbank auch ein anderer, z. B. handelsüblicher Vektor wie pHC79 (Hohn und Collins, Gene 11 (1980) 291) verwendet werden.

Beispiel 3:

Erstellung der Genbank

DNA von Alcaligenes faecalis wird nach der für eukaryontische DNA beschriebenen Methode (Maniatis et al., Molecular Cloning, A Laboratory Manual (1982), Seite 280 bis 285) isoliert und mit Sau3AI partiell gespalten. Der Vektor pOCAM 12 wird mit BamHI verdaut und mit DNA-Fragmenten der Größe von etwa 25-40 kb ligiert. Die Ligierung und die Verpackung in λ-Phagen erfolgt nach den Angaben des Herstellers (Amersham: in vitro packaging system for Lambda DNA, Code No. 334Z) bzw. entsprechend Maniatis et al., Seite 296 bis 299.

Beispiel 4:

Infektion des E. coli-Indikatorstamms DH1 und "Screening"

Bakterien des Stamms E. coli DH1 werden in LBMM-Medium (LB-Medium und zusätzlich 2 g Maltose sowie 2,5 g Magnesiumsulfat-Heptahydrat pro Liter) auf eine optische Dichte ($OD_{550}$) von etwa 1 angezogen. 200 $\mu$l dieser Suspension werden mit bis zu 50 $\mu$l Phagen-Suspension vermischt, 30 min bei 37°C inkubiert, 1 ml LBMM zugegeben und weitere 50 min inkubiert. Aliquots von 100 $\mu$l werden auf LB-Platten, supplementiert mit 10 $\mu$g Tetracyclin/ml, ausgestrichen. Einzelne Kolonien werden ausgestochen, in 5 ml-Kulturen angezogen und wie oben beschrieben in 5er-Gruppen untersucht. Nach Überprüfung von 2 700 Cosmidklonen zeigt ein untersuchter Pool im Autoradiogramm das erwartete Signal in der Position von N-Acetyl-PTC. Die zu diesem Pool gehörenden Klone werden einzeln im Modifikationstest untersucht und der für die Modifikation verantwortliche Klon identifiziert.

Der gefundene Klon besitzt ein Insert von etwa 25 000 bis 30 000 bp. Er verleiht Bakterien des Stammes E. coli DH1 und der Arten Agrobacterium tumefaciens und Rhizobium meliloti eine Resistenz bis zu etwa 50 mM PTC.

Durch Restriktionsanalyse kann die enzymatische Aktivität auf ein etwa 2 kb BstEII-BglII-Fragment des Inserts eingegrenzt werden. Dieses Fragment ist durch die Restriktionskarte (Figur 1) charakterisiert. Durch weitere Eingrenzung und Sequenzierung wird das Phosphinothricin-Resistenzgen charakterisiert, dessen DNA-Sequenz in der Tabelle 1 wiedergegeben ist.

Tabelle 1

```
  1-   Met Pro Ser Ser Ser Ser His Pro Ser Thr Pro Asp Ala Pro Gln
  1-   ATG CCG TCA TCT TCG TCT CAC CCC TCC ACT CCC GAC GCG CCG CAA

 16-   Arg Val Gly Val Glu Leu Ala Arg Cys Ala Cys Thr Val Arg Val
 46-   CGC GTC GGC GTC GAA CTG GCG CGT TGC GCA TGC ACG GTG CGC GTC

 31-   Val Arg Asp Asp Asp Leu Pro Ala Ile Thr Ala Ile Tyr Ala His
 91-   GTG CGT GAC GAC GAC CTG CCC GCC ATC ACG GCC ATC TAC GCC CAT

 46-   His Val Arg Thr Gly Thr Ala Ser Phe Glu Glu Val Pro Pro Asp
136-   CAC GTG CGT ACC GGC ACG GCA TCG TTC GAA GAG GTG CCA CCC GAC

 61-   Asp Thr Glu Met Arg Ala Arg Cys Ala Lys Val Leu Asp Ala Gly
181-   GAC ACC GAG ATG CGC GCG CGT TGC GCC AAG GTA CTC GAC GCC GGA

 76-   Leu Pro Tyr Leu Val Ala Glu Arg Asp Gly Lys Leu Leu Gly Tyr
226-   CTG CCG TAT CTC GTC GCC GAA CGC GAC GGC AAG CTG CTC GGC TAC

 91-   Ala Tyr Ala Thr His Tyr Arg Pro Arg Ser Ala Tyr Arg Phe Thr
271-   GCA TAC GCC ACG CAT TAC CGG CCG CGC TCC GCC TAC CGT TTC ACG

106-   Leu Glu Asp Ser Val Tyr Ile Ala Pro Asp Ala Ile Gly Gln Gly
316-   CTG GAA GAC TCG GTG TAT ATC GCC CCC GAT GCG ATC GGG CAG GGC

121-   Val Gly Arg Thr Leu Leu Leu Thr Leu Ile Ala Arg Cys Glu Gly
361-   GTA GGG CGC ACG CTG TTG CTC ACG CTC ATC GCG CGT TGC GAA GGC

136-   Gly Pro Trp Arg Gln Leu Ile Ala Asn Val Gly Asp Ser Gly Asn
406-   GGC CCG TGG CGG CAA CTG ATT GCG AAC GTC GGC GAC AGC GGC AAT

151-   Thr Ala Ser Leu Gly Leu His Ala Ala Cys Gly Phe Val Gln Ala
451-   ACG GCG TCC CTC GGT CTG CAT GCC GCC TGC GGC TTC GTG CAG GCA

166-   Gly Val Leu Lys Ser Val Gly Phe Lys Phe Gly Arg Trp Ile Asp
496-   GGC GTG CTC AAG TCC GTC GGC TTC AAG TTC GGC CGC TGG ATC GAC

181-   Thr Val Leu Met Gln Arg Pro Leu Asn Ala Gly Asp Thr Thr Leu
541-   ACG GTG CTC ATG CAA CGG CCG CTC AAC GCG GGC GAC ACA ACG CTG

196-   Pro Glu
586-   CCG GAG TAA
```

Patentansprüche
Patentansprüche für folgende Vertragsstaaten : CH, DE, FR, GB, IT, LI, NL

1. Resistenzgen gegen Phosphinothricin (PTC), erhältlich durch Selektion nicht-pilzähnlicher Bakterien auf PTC-Resistenz, Gewinnung der DNA, Anlegen einer Genbank, Isolierung PTC-resistenter Klone und Gewinnung des PTC-Resistenzgens aus diesen Klonen.

2. Gen nach Anspruch 1, dadurch gekennzeichnet, daß man Bakterien der Gattung Pseudomonas, Alcaligenes, Agrobacterium, Enterobacter, Serratia oder Cedecea einsetzt, vorzugsweise Bakterien der Art Pseudomonas paucimobilis, Alcaligenes faecalis oder eutrophus, Agrobacterium tumefaciens, Enterobacter agglomerans, Serratia plymuthica oder Cedecea Gr. V einsetzt.

3. Gen nach einem der vorhergehenden Ansprüche, erhältlich aus der Gesamt-DNA von auf PTC-Resistenz selektierten nicht-pilzähnlichen Bakterien, vorzugsweise aus Alcaligenes faecalis, durch Schneiden mit BstEII und Bgl II, Klonieren eines 2 kb großen Fragments und Selektion auf PTC-Resistenz.

4. Gen nach Anspruch 3, gekennzeichnet durch die Restriktionskarte gemäß Figur 1.

5. Resistenzgen gegen PTC, gekennzeichnet durch die DNA-Sequenz der Tabelle 1.

6. Verwendung des dem Gen nach einem oder mehreren der Ansprüche 1 bis 4 zugrundeliegenden Strukturgens oder des Gens nach Anspruch 5 zur Herstellung PTC-resistenter Pflanzen.

7. Verwendung des Gens nach einem oder mehreren der Ansprüche 1 bis 5 als PTC-Resistenzmarker in Bakterien oder Pflanzenzellen.

8. Nutzpflanzen und deren Teile, gekennzeichnet durch ein exprimierbares Resistenzgen nach einem oder mehreren der Ansprüche 1 bis 5.

9. Vermehrungsgut von Nutzpflanzen, gekennzeichnet durch ein exprimierbares Resistenzgen nach einem oder mehreren der Ansprüche 1 bis 5.

10. Verwendung von Mikroorganismen-Populationen, die das Resistenzgen nach einem oder mehreren der Ansprüche 1 bis 5 exprimieren, in Kläranlagen oder auf landwirtschaftlichen Nutzflächen.

**Patentansprüche für den folgenden Vertragsstaat : ES**

1. Verfahren zur Gewinnung eines Resistenzgens gegen Phosphinothricin (PTC), dadurch gekennzeichnet, daß man nicht-pilzähnliche Bakterien auf PTC-Resistenz selektiert, die DNA der Selektanten isoliert, daraus eine Genbank anlegt, PTC-resistente Klone isoliert und aus deren DNA das Resistenzgen gewinnt.

2. Verfahren nach Anspruch 1, dadurch gekennzeichnet, daß man Bakterien der Gattung Pseudomonas, Alcaligenes, Agrobacterium, Enterobacter, Serratia oder Cedecea einsetzt.

3. Verfahren nach Anspruch 2, dadurch gekennzeichnet, daß man Bakterien der Art Pseudomonas paucimobilis, Alcaligenes faecalis oder eutrophus, Agrobacterium tumefaciens, Enterobacter agglomerans, Serratia plymuthica oder Cedecea Gr. V einsetzt.

4. Verfahren nach Anspruch 1, dadurch gekennzeichnet, daß man Bakterien der Art Alcaligenes faecalis einsetzt, die DNA von PTC-resistenten Selektanten mit BstEII und BglII schneidet, ein 2 kb großes Fragment kloniert und auf PTC-Resistenz selektiert.

5. Verfahren zur Herstellung eines Resistenzgens gegen PTC, dadurch gekennzeichnet, daß man eine DNA gemäß Tabelle 1 chemisch synthetisiert.

6. Verfahren zur Herstellung von PTC-resistenten Pflanzenzellen, Pflanzen, Pflanzenteilen und Vermehrungsgut, dadurch gekennzeichnet, daß man in die Pflanzenzellen ein exprimierbares Gen einbringt, das nach Anspruch 1 bis 5 erhalten wurde.

7. Verfahren zur Selektion von Pflanzen, Pflanzenzellen oder Bakterien, dadurch gekennzeichnet, daß man in die Zellen ein nach Anspruch 1 bis 5 erhaltenes exprimierbares Gen einbringt und auf PTC-Resistenz selektiert.

8. Verfahren zum biologischen Abbau von PTC in PTC enthaltenden Rückständen, landwirtschaftlichen Nutzflächen und Abwässern, dadurch gekennzeichnet, daß man die Rückstände oder Abwässer mit Mikroorganismen-Populationen in Kontakt bringt, die ein nach Anspruch 1 bis 5 erhaltenes Gen exprimieren.

**Claims**

**Claims for the following Contracting States : CH, DE, FR, GB, IT, LI, NL**

1.  A phosphinothricin(PTC)-resistance gene, obtainable by selection of bacteria, which are not fungus-like, for PTC resistance, extraction of the DNA, construction of a gene bank, isolation of PTC-resistant clones, and extraction of the PTC-resistance gene from these clones.

2.  A gene as claimed in claim 1, wherein bacteria of the genus Pseudomonas, Alcaligenes, Agrobacterium, Enterobacter, Serratia or Cedecea bacteria of the species Pseudomonas paucimobilis, Alcaligenes faecalis or eutrophus, Agrobacterium tumefaciens, Enterobacter agglomerans, Serratia plymuthica or Cedecea Gr. V are used.

3.  A gene as claimed in one of the preceding claims, obtainable from the complete DNA from bacteria which are not fungus-like and have been selected for PTC resistance, preferably from Alcaligenes faecalis, by cutting with BstEII and Bgl II, cloning of a fragment which is 2 kb in size, and selecting for PTC resistance.

4.  A gene as claimed in claim 3, which has the restriction map shown in Figure 1.

5.  A PTC-resistance gene which has the DNA sequence in Table 1.

6.  The use of the structural gene which forms the basis for the gene as claimed in one or more of claims 1 to 4, or of the gene as claimed in claim 5, for producing PTC-resistant plants.

7.  The use of the gene as claimed in one or more of claims 1 to 5, as marker of PTC resistance in bacteria or plant cells.

8.  Useful plants and parts thereof, which harbor an expressible resistance gene as claimed in one or more of claims 1 to 5.

9.  Propagation material from useful plants, which harbors an expressible resistance gene as claimed in one or more of claims 1 to 5.

10. The use of microorganism populations which express the resistance gene as claimed in one or more of claims 1 to 5 in sewage treatment plants or on areas under agricultural use.

**Claims for the following Contracting State : ES**

1.  A process for obtaining a phosphinothricin(PTC)-resistance gene, which comprises selecting bacteria which are not fungus-like for PTC resistance, isolating the DNA from the selectants, constructing a gene bank therefrom, isolating PTC-resistant clones, and obtaining the resistance gene from the DNA thereof.

2.  The process as claimed in claim 1, wherein bacteria of the genus Pseudomonas, Alcaligenes, Agrobacterium, Enterobacter, Serratia or Cedecea are used.

3.  The process as claimed in claim 2, wherein bacteria of the species Pseudomonas paucimobilis, Alcaligenes faecalis or eutrophus, Agrobacterium tumefaciens, Enterobacter agglomerans, Serratia plymuthica or Cedecea Gr. V are used.

4.  The process as claimed in claim 1, wherein bacteria of the species Alcaligenes Faecalis are used, the DNA from PTC-resistant selectants is cut with BstEII and BglIII, and a fragment which is 2 kb in size is cloned and selected for PTC resistance.

5.  A process for the preparation of a PTC-resistance gene, which comprises chemical synthesis of the DNA shown in Table 1.

6.  A process for the production of PTC-resistant plant cells, plants, parts of plant and propagation material, which comprises inserting into the plant cells an expressible gene which has been obtained as claimed in claim 1 to 5.

7. A process for the selection of plants, plant cells or bacteria, which comprises inserting into the cells an expressible gene which has been obtained as claimed in claim 1 to 5, and selection for PTC resistance.

8. A process for the biological degradation of PTC in PTC-containing residues, areas under agricultural use, and sewage, which comprises contacting the residues or sewage with microorganism populations which express a gene obtained as claimed in claim 1 to 5.

**Revendications**
**Revendications pour les Etats contractants suivants : CH, DE, FR, GB, IT, LI, NL**

1. Gène de résistance à la phosphinothricine (PTC), obtenu par sélection de bactéries non fongiques pour leur résistance à la PTC, obtention de l'ADN, édification d'une banque de gènes, isolement des clones résistant à la PTC et obtention du gène de résistance à la PTC à partir de ces clones.

2. Gène selon la revendication 1, caractérisé en ce qu'on utilise des bactéries du genre Pseudomonas, Alcaligenes, Agrobacterium, Enterobacter, Serratia ou Cedecea, de préférence des bactéries de l'espèce Pseudomonas paucimobilis, Alcaligenes faecalis ou eutrophus, Agrobacterium tumefaciens, Enterobacter agglomerans, Serratia Plymuthica ou Cedecea Gr. V.

3. Gène selon une des revendications précédentes, obtenu à partir de l'ADN total de bactéries non fongiques sélectionnées pour leur résistance à la PTC, de préférence à partir d'Alcaligenes faecalis, par coupure avec BstEII et BglII, clonage d'un fragment de 2 kb et sélection pour sa résistance à la PTC.

4. Gène selon la revendication 3, caractérisé par la carte de restriction représentée sur la Figure 1.

5. Gène de résistance à la PTC, caractérisé par la séquence d'ADN du Tableau 1.

6. Utilisation du gène structural qui est à la base du gène selon une ou plusieurs des revendications 1 à 4 ou du gène selon la revendication 5, pour la fabrication de plantes résistantes à la PTC.

7. Utilisation du gène selon une ou plusieurs des revendications 1 à 5 comme marqueur de résistance à la PTC dans les bactéries ou les cellules végétales.

8. Plantes utilitaires et parties de ces plantes, caractérisées par un gène de résistance exprimable obtenu selon une ou plusieurs des revendications 1 à 5.

9. Matériau de reproduction des plantes utilitaires, caractérisé par un gène de résistance exprimable obtenu selon une ou plusieurs des revendications 1 à 5.

10. Utilisation de populations de microorganismes, qui expriment le gène de résistance selon une ou plusieurs des revendications 1 à 5, dans des installations d'épuration ou sur des surfaces utilitaires agricoles.

**Revendications pour l'Etat contractant suivant : ES**

1. Procédé pour l'obtention d'un gène de résistance à la phosphinothricine (PTC), caractérisé en ce qu'on sélectionne des bactéries non fongiques pour leur résistance à la PTC, on isole l'ADN des sélectants, à partir duquel on édifie une banque de gènes, on isole les clones résistants à la PTC et on obtient le gène de résistance à partir de l'ADN de ces clones.

2. Procédé selon la revendication 1, caractérisé en ce qu'on utilise des bactéries du genre Pseudomonas, Alcaligenes, Agrobacterium, Enterobacter, Serratia ou Cedecea.

3. Procédé selon la revendication 2, caractérisé en ce qu'on utilise des bactéries de l'espèce Pseudomonas paucimobilis, Alcaligenes faecalis ou eutrophus, Agrobacterium tumefaciens, Enterobacter agglomerans, Serratia plymuthica ou Cedecea Gr. V.

4. Procédé selon la revendication 1, caractérisé en ce qu'on utilise des bactéries de l'espèce Alcaligenes faecalis, on coupe, avec BstEII et BglII, l'ADN des sélectants résistants à la PTC, on clone un fragment de taille 2 kb et on effectue une sélection pour sa résistance à la PTC.

5. Procédé de fabrication d'un gène de résistance à la PTC, caractérisé en ce qu'on synthétise chimiquement un ADN selon le Tableau 1.

6. Procédé de fabrication de cellules végétales, de plantes, de parties de plantes et de matériau de reproduction, résistants à la PTC, caractérisé en ce qu'on introduit dans les cellules végétales un gène exprimable obtenu selon une des revendications 1 à 5.

7. Procédé de sélection de plantes, de cellules végétales ou de bactéries, caractérisé en ce qu'on introduit dans les cellules un gène exprimable obtenu selon une des revendications 1 à 5 et qu'on procède à une sélection pour déterminer leur résistance à la PTC.

8. Procédé de dégradation biologique de la PTC dans des résidus contenant de la PTC, des surfaces utilitaires agricoles et des eaux usées, caractérisé en ce qu'on met les résidus ou eaux usées en contact avec des populations de microorganismes, qui expriment le gène obtenu selon une des revendications 1 à 5.

# FIG.1

# FIG.2

pOCAM 12
8,5 kb